(19) Europäisches Patentamt
European Patent Office
Office européen des brevets

(11) **EP 4 549 944 A1**

## (12) EUROPEAN PATENT APPLICATION

(43) Date of publication:
**07.05.2025 Bulletin 2025/19**

(21) Application number: **23461675.3**

(22) Date of filing: **30.10.2023**

(51) International Patent Classification (IPC):
*G01N 33/68* (2006.01)   *A61B 5/00* (2006.01)
*A61B 10/00* (2006.01)   *G01N 15/1433* (2024.01)

(52) Cooperative Patent Classification (CPC):
**G01N 33/68; A61B 10/0058; G01N 15/1433**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Checkcells, Inc.**
**Santa Monica, CA 90401 (US)**

(72) Inventors:
• **KOSELA, Marcin**
**Santa Monica, CA 90401 (US)**

• **ASZYK, Jakub**
**Santa Monica, CA 90401 (US)**
• **JAREK, Mateusz**
**Santa Monica, CA 90401 (US)**
• **KLIMEK, Jakub**
**Santa Monica, CA 90401 (US)**
• **PROKOP, Tomasz**
**Santa Monica, CA 90401 (US)**

(74) Representative: **Boult Wade Tennant LLP**
**Salisbury Square House**
**8 Salisbury Square**
**London EC4Y 8AP (GB)**

## (54) METHODS, SYSTEMS AND DEVICES FOR TESTING BIOLOGICAL SAMPLE

(57) The invention relates to methods, systems, devices and kits for testing biological sample, preferably semen. Preferably, the methods, systems, devices and kits of the invention are used in point-of-care determination of parameters of cells contained in a biological sample. The invention also relates to uses of the systems, devices and kits for diagnostic purposes, preferably for diagnosis of diseases associated with or resulting in male infertility.

EP 4 549 944 A1

**Description**

**FIELD OF THE INVENTION**

The invention relates to methods, systems, devices and kits for testing biological sample, preferably semen. Preferably, the methods, systems, devices and kits of the invention are used in point-of-care determination of parameters of cells contained in a biological sample. The invention also relates to uses of the systems, devices and kits for diagnostic purposes, preferably for diagnosis of diseases associated with or resulting in male infertility.

**BACKGROUND TO THE INVENTION**

Different fertility tests are available to male patients in need of checking their fertility status.

Patients can attend a clinic for an in-person sample collection, which is followed by a time-consuming analysis of the sample in a laboratory by trained personnel. This approach typically delivers results which meet World Health Organization (WHO) standard (see "WHO laboratory manual for the examination and processing of human semen"; sixth edition, WHO, 2021) and leads to a comprehensive understanding of vital sperm parameters, such as total sperm count, concentration, morphology, motility, vitality, volume and pH. However, this approach is associated with many drawbacks. Firstly, the patient is required at the clinic for sample collection, which is associated with a level of embarrassment for some, and it is time consuming. Secondly, obtaining results from a laboratory testing typically takes a few days to few weeks; therefore this approach is not suitable for daily testing of sperm fertility, for example, pre-IVF treatment, in traditional pregnancy planning, postvasectomy or post-reverse vasectomy. The laboratory testing also involves manual examination of the sample under a microscope, which is prone to human error.

A number of use-at-home devices for testing semen is available. For example, US Patent no. 5,605,803 describes a monoclonal antibody based lateral flow device which provides information in relation to sperm count. International application no. WO2019048018 A1 describes a device for testing the quality of semen, in cooperation with an electronic apparatus, the device being able to output the sperm motility and concentration. However, these devices fail to provide results in relation to other parameters, such as morphology, vitality or pH, which are crucial for understanding the overall quality of the sample.

An in-between approach relies on the collection of the semen sample at home, which saves patient some time and potential embarrassment, followed by sending the sample to a laboratory for analysis. Although this approach remedies the drawbacks of attending an appointment at the clinic, the time it takes to obtain the results is measured in days; therefore this approach is also not suitable for daily testing. In addition, obtaining comprehensive results in relation to the fertility status of a patent based on a sample that was analysed in a laboratory is typically very expensive.

Sperm levels can be impacted by a variety of factors ranging from diet, weight, and stress levels, to hormone imbalances, medications, or underlying health conditions. Thus, a need exists for a point-of-care (i.e. not requiring laboratory analysis) methods and devices which provide a comprehensive panel of markers of the tested sample within a manner of minutes and for a reasonable price so that testing can be performed daily. A need also exists for a point-of-care (i.e. not requiring laboratory analysis) methods and devices which provide high quality results in relation to at least three parameters of the tested sample.

**SUMMARY OF THE INVENTION**

In a first aspect, the present invention provides a method for determination of at least three parameters of cells contained in a biological sample, preferably semen, wherein the method comprises the steps of:

(a) providing a first, second and third portion of the biological sample;
(b) recording with a camera (i) a first video of the first portion of the biological sample; (ii) a second video of the second portion of the biological sample; and (iii) a third video of the third portion of the biological sample; and
(c) analysing the first, second, and third recorded videos of the biological sample to determine at least three parameters of the cells in the biological sample, wherein the at least three parameters are selected from morphology, vitality, pH, total count, concentration and motility.

In a further aspect, the invention provides a method for point-of-care determination of at least three parameters of cells contained in a biological sample, preferably semen, wherein the method comprises the steps of:

(a) combining (i) a first portion of the biological sample with a vitality reagent; and (ii) a second portion of the biological sample with a pH reagent;
(b) recording with a camera (i) a first video of the first portion of the biological sample; (ii) a second video of the second portion of the biological sample; and (iii) a third video of a third portion of the biological sample; and

(c) analysing the first, second, and third recorded videos of the biological sample to determine at least three parameters of the cells in the biological sample,

wherein the at least three parameters are selected from morphology, vitality, pH, total count, concentration and motility, preferably wherein at least two of the at least three parameters are pH and vitality.

In a further aspect, the invention provides a system for analysis of at least one parameter of a cell contained in a biological sample, wherein the biological sample is preferably semen, the system comprising:

a device comprising:

(i) a camera configured to record at least three videos of the biological sample; and
(ii) at least one processor communicatively connected to the camera, the at least one processor being configured to:

a) receive the at least three recorded videos of the biological sample captured by the camera;
b) transmit, preferably via a Wi-Fi module, the at least three recorded videos of the biological sample to an electronic apparatus;

the electronic apparatus configured to receive the at least three recorded videos of the biological sample, and to:

a) return distribution of motility of a cell in the biological sample, wherein the distribution of motility is obtained from the average velocity of the cell; and/or
b) return morphology of a cell, wherein morphology of the cell categorizes the cell as normal or abnormal;

and wherein the at least one parameter is selected from morphology and motility.

Preferably, the system analyses at least three parameters of a cell contained in a biological sample, wherein at least two of the at least three parameters are morphology and motility.

In a further aspect, the invention provides a method for testing the quality of a biological sample, preferably semen, the method comprising the steps:

(a) recording a video of the biological sample with a camera;
(b) identifying a plurality of cells in the biological sample based on the recorded video;
(c) determining a location of a first cell of the plurality of biological cells in a first frame of the recorded video; determining a location of the first cell of the plurality of biological cells in a second frame of the recorded video; and determining a location of the first cell of the plurality of biological cells in a third frame of the recorded video;
(d) obtaining an average velocity of the first cell based on the location of the first cell in the first, second and third from of the recorded video.

In a further aspect, the invention provides a device for analysis of a biological sample, preferably semen, wherein the device is preferably used with an electrical apparatus, wherein the device comprises:

(a) a port configured to receive a solid support containing a biological sample;
(b) a light source configured to direct a light beam onto the solid support received in the port;
(c) an optical system comprising at least one lens, wherein the optical system is configured to magnify an image of the biological sample contained in or on the solid support;
(d) an image processing system comprising (i) a camera configured to record at least one video of the magnified biological sample; and (ii) an image processor configured to transmit, preferably via a Wi-Fi module, the at least one video of the magnified biological sample to the electronic apparatus.

In a further aspect, the invention provides a device for analysis of a biological sample, wherein the device is preferably used with an electrical apparatus, wherein the device comprises:

(a) a port configured to receive a solid support containing a biological sample;
(b) a light source configured to direct a light beam onto the solid support received in the port;
(c) an optical system comprising means for converting phase shifts in the light beam passing through the biological sample to brightness changes;

(d) an image processing system comprising (i) a camera configured to record at least one video of the biological sample; and (ii) an image processor configured to transmit, preferably via a Wi-Fi module, the at least one video of the biological sample to the electronic apparatus.

Preferably, in all aspects of the invention, the methods, systems, devices and kits are point-of-care methods, systems, devices and kits. Preferably, the methods, systems, devices and kits of the invention can be performed and/or used without trained personnel (e.g. a lab technician). More preferably still, methods, systems, devices and kits of the invention do not require laboratory analysis in order to obtain a result.

Preferably, the methods, devices, systems and kits of the invention are suitable for determining at least 4 parameters of cells contained in a biological sample. More preferably still, the methods, devices, systems and kits of the invention are suitable for determining at least 6 parameters of cells contained in a biological sample. Preferably, the at least 6 parameters are total count, concentration, pH, motility, vitality and morphology. The methods and kits of the invention also provide methods and means for assessing the volume of the biological sample.

Preferably, the optical system is configured to magnify an image of the biological sample at least 100 times. More preferably still, the optical system is configured to magnify an image of the biological sample at least 200 times or at least 400 times. Preferably, the optical system comprises two lenses.

The devices or systems of the invention allow to obtain a video or an image of high resolution. Preferably, the resolution is at least 200 nm. More preferably still, the resolution is at least 50 nm.

In an aspect, the invention provides a device for point-of-care analysis of the quality of a biological sample, wherein the device is preferably used with an electrical apparatus, the device comprising:

(a) a port configured to receive a solid support containing a biological sample;
(b) a light source configured to direct a light beam onto the solid support received in the port;
(c) an optical system comprising at least one lens, wherein the optical system is configured to magnify an image of the biological sample contained in or on the solid support;
(d) an image processing system comprising (i) a camera configured to record at least one video of the magnified biological sample; and (ii) an image processor configured to transmit, preferably via a Wi-Fi module, the at least one video of the magnified biological sample to the electronic apparatus.

In an aspect, the invention provides a device for point-of-care determination of at least three parameters of cells contained in a biological sample, wherein the device is preferably used with an electrical apparatus, the device comprising:

(a) a port configured to receive a solid support containing a biological sample;
(b) a light source configured to direct a light beam onto the solid support received in the port;
(c) an optical system comprising at least one lens, wherein the optical system is configured to magnify an image of the biological sample contained in or on the solid support;
(d) an image processing system comprising (i) a camera configured to record at least one video of the magnified biological sample; and (ii) an image processor configured to transmit, preferably via a Wi-Fi module, the at least one video of the magnified biological sample to the electronic apparatus.

In a further aspect, the invention provides a kit for analysis of the biological sample, the kit comprising:

(a) the device as described herein;
(b) a solid support; and
(c) one or more reagents for combining with the biological sample.

In an aspect, the invention provides a test kit for use with the device as described herein or the system as described herein, the test kit comprising:

(a) a solid support; and
(b) one or more reagents for combining with the biological sample.

In a further aspect, the invention provides a use of the system as described herein, the kit as described herein, or the device as described herein for testing the quality of a biological sample, preferably semen.

In a further aspect, the invention provides a use of the system as described herein, the kit as described herein, or the device as described herein for diagnosing a disease associated with or resulting in male infertility.

In a further aspect, the invention provides a use of the system as described herein, the kit as described herein, or the device as described herein for assessing male fertility.

In a further aspect, the invention provides a use of the system as described herein, the kit as described herein, or the device as described herein for testing a biological sample, preferably semen, prior to in vitro fertilization (IVF).

In a further aspect, the invention provides a use of the system as described herein, the kit as described herein, or the device as described herein for testing a biological sample, preferably semen, after reverse-vasectomy.

In a further aspect, the invention provides a use of the system as described herein, the kit as described herein, or the device as described herein in assessing the fertility status of an animal.

In a further aspect, the invention provides a use of the system as described herein, the kit as described herein, or the device as described herein in veterinary diagnostics.

Each aspect or embodiment as defined herein may be combined with any other aspect(s) or embodiment(s) unless clearly indicated to the contrary. In particular any feature indicated as being preferred or advantageous may be combined with any other feature or features indicated as being preferred or advantageous.

The present inventors have surprisingly discovered methods, systems, devices and kits for testing different parameters of a biological sample in a point-of-care setting. Due to a portable nature of the invention, the methods, systems, devices and kits are particularly suitable for use at patient's home or on farms (if animals are being tested). Moreover, the inventors of the present invention have optimised sample preparation methods, different elements of the device and the processing of the sample videos or images by a software to accomplish a testing devices and methods which allow to deliver comprehensive, high quality results within a manner of minutes and without any laboratory equipment or analysis. The methods, devices, systems and kits of the invention provide an additional advantage of not requiring handling by trained personnel making them particularly suitable for use by the patients themselves (or their carers). The present invention provides further benefit of not requiring the patent to attend a clinic in order to produce a sample.

## BRIEF DESCRIPTION OF THE DRAWINGS

These and other aspects of the invention will now be described with reference to the accompanying Figures, in which:

Fig. 1 illustrates one embodiment of the device aspect of the invention.

Fig. 2 illustrates another embodiment of the device aspect of the invention comprising means for adjusting focus.

Fig. 3 illustrates exemplary workflow of the steps taken in the determination of average velocities of the cells in the biological sample.

Fig. 4 illustrates examples of abnormal cell (e.g. sperm) categories used in some embodiments of cell morphology determination.

Fig. 5 illustrates cell images obtained using the present invention (A) and prior art devices (B).

Fig. 6 illustrates an exemplary histogram showing distribution of cell motility.

Fig. 7 illustrates another embodiment of the device aspect of the invention comprising elements of the phase contrast microscope.

## DETAILED DESCRIPTION OF THE INVENTION

Unless otherwise defined herein, scientific, and technical terms used in connection with the present invention shall have the meanings that are commonly understood by those of ordinary skill in the art. The meaning and scope of the terms should be clear, however, in the event of any latent ambiguity, definitions provided herein take precedent over any dictionary or extrinsic definition.

It should be understood that singular prepositions such as "a," "an," and "the," are often used for convenience, however, all instances of the singular are intended to encompass the plural unless otherwise indicated either explicitly or from context. The terms "comprising", "including" and "having" are intended to be inclusive and mean that there may be additional elements other than the listed elements. Further, it should be understood that all references, including journal articles, books, patents, technical documents, and the like, mentioned in this disclosure are hereby incorporated by reference in their entirety and for all purposes.

The term "about" as used herein for numerical data refers to a value within 10% of the underlying parameter (i.e., plus or minus 10%).

The expression "determining motility of a cell" (or similar) as used herein is understood to include (i) identifying at least one cell (preferably a plurality of cells) in multiple frames of a recorded video (or multiple images of a series of images taken), (ii) tracking the cell (preferably the plurality of cells) through multiple frames of the recorded video (or multiple images of a series of images taken) to determine the velocities of the cell (preferably the plurality of cells) and (iii) obtaining an average velocity of the cell (preferably each cell from the plurality of cells) from the determined velocities in (ii). The motility of the cell may be determined from a video that is at least 1 second long. Preferably, the video is at least 2 seconds, 5 seconds, or 10 seconds long. Preferably, the multiple frames of a recorded video comprise at least 24, at least 25, at least 30, at least 50, at least 60, at least 120, or at least 240 frames. The image sensor (e.g. a camera) may record at least 24 frames per second, at least 25 frames per second, at least 30 frames per second, at least 50 frames per second, at least 60 frames per second, at least 120 frames per second, or at least 240 frames per second, Thus, the step of tracking the cell (preferably the plurality of cells) through multiple frames of the recorded video (or multiple images of a series of images taken) to determine the velocities of the cell (preferably the plurality of cells) may comprise determining the velocities of the cell (preferably the plurality of cells) at an interval of 1/24 second, 1/25 second, 1/30 second, 1/50 second, 1/60 second, 1/120 second, or 1/240 second. The cell may be identified in n number of frames. The $n$ number may be any number between 24 and 240. The cell may be tracked between the first frame and the $n^{th}$ frame. The average velocity may be determined based on the distance travelled by the cell between the first cell and the $n^{th}$ cell. Preferably, the motility of the cell (or plurality of cells) is outputted (e.g. in the electronic apparatus) as distribution of the cell motility categories (immotile, rapidly progressive, slowly progressive, non-progressive). Thus, for example, if 10 cells were examined and 5 of the cells were found to be rapidly progressive, 4 of the cells were found to be slowly progressive and 1 cell was found to be immotile, the distribution resulting from the analysis would be: 5 (rapidly progressive) : 4 (slowly progressive) : 1 (immotile) : 0 (non-progressive). The distribution of motility may be the result of the test performed by a user (e.g. a patient) of the systems, devices or kits described herein. The result of the test may be outputted on an electronic apparatus, such as a smartphone or a tablet.

The expression "determining morphology of a cell" as used herein is understood to include the steps (i) identifying a cell (or cells) in a frame of the recorded video (or in an image of the series of images taken), (ii) applying an image classification algorithm to classify the cell (or cells) as either normal (i.e. healthy) or abnormal (i.e. unhealthy), or as belonging to one of the several classes of cells comprising defects. To increase percentage certainty of belonging to a given class, the cell might be analysed based on the image of the cell from different frames of the recorded video.

The expression "determining vitality of a cell" as used herein is understood to include the steps (i) identifying a cell (or cells) in a frame of the recorded video (or in an image of the series of images taken), (ii) applying an image classification algorithm to classify the cell (or cells) as either live or dead based on the staining pattern.

The expression "determining the concentration" (or similar) as used herein is understood as detecting the number of cells in a single frame of the recorded video (or in an image of a series of images taken) and dividing this number by the volume of the observed frame. The volume of the observed frame is computed as the thickness of the sample layer (which is known from the height of the solid support chamber and/or applied volume). Typically, the concentration is averaged over all recorded frames (or images taken). The expression "total cell count" is determined from cell concentration multiplied by the total volume of the collected sample.

The term "point-of-care" as used herein in the context of methods, devices, systems and kits is intended to encompass any methods, devices, systems and kits which do not require analysis of the results in/by a laboratory and/or by trained personnel. Typically, the point-of-care methods, systems, devices or kits are used in the patient home. Embodiments in which the point-of-care methods, devices, systems and kits are used at different locations, such as medical testing facilities, clinics, hospitals, farms or pharmacies, are also envisaged provided that the analysis of the results is not performed in/by a laboratory and/or by trained personnel. The use of point-of-care methods, systems, devices and kits of the present invention benefits the patient by delivering a quick answer, and optionally putting them on an immediate treatment plan. Likewise there would be potential benefit to the healthcare provider, by avoiding outsourcing to a lab and the resource and time costs involved and keeping the patient time down to a single session (if necessary) in the first instance, or avoiding a visit to the doctors entirely.

The term "portion of the biological sample" as used herein is understood to mean a part of the collected sample. For example, in the context of a sperm sample, the total sample ejaculated may be collected in a container, and from the container, a portion of the sample may be taken for further analysis and/or staining. The portion of the biological sample may be less than 80%, less than 70%, less than 60%, less than 50%, less than 40%, less than 30%, less than 20%, less than 10%, less than 5% or less than 1% of the total sample collected.

Manual microscopy-based testing and computer-assisted semen analysis (CASA) systems are the current standard methods to measure semen quality, but these methods are labour-intensive, expensive, and laboratory-based. The CASA technique requires highly trained technicians for producing reliable and repeatable results. It also requires bulky microscopy-based image analysis systems that significantly limit its point-of-care applications in clinical settings, stud farming, and animal breeding. The vast majority of fertility clinics and small hospitals do not possess CASA platforms available in the market and use a less accurate and subjective manual method for semen analysis. Manual test results are subjective making it difficult to compare results from different clinics.

The present invention overcomes the above problems by providing point-of-care devices, systems and kits which include a combination of features that allow obtaining comprehensive high quality results for determining the quality of a biological sample without a need to perform laboratory analysis, or manual analysis by the user or by trained personnel.

The present invention provides a method for determination of at least three parameters of cells contained in a biological sample, wherein the method comprises the steps of:

(a) providing a first, second and third portion of the biological sample;

(b) recording with an image sensor (i) a first video of the first portion of the biological sample; (ii) a second video of the second portion of the biological sample; and (iii) a third video of the third portion of the biological sample; and

(c) analysing the first, second, and third recorded videos of the biological sample to determine at least three parameters of the cells in the biological sample, wherein the at least three parameters are selected from morphology, vitality, pH, total count, concentration and motility.

The method may comprise a step of combining at least one of the portions of the biological sample with a reagent prior to the step of recording. The use of the reagent may facilitate determination of at least one of the parameters of the cells contained in the biological sample. Preferably, the method comprises a step of combining at least two portions of the biological sample with two different reagents prior to the step of recording. For example, the method may comprise combining a first portion of the biological sample with a vitality reagent. The vitality reagent may facilitate distinction of living cells from dead cells. The method may comprise combining a second portion of the biological sample with a pH reagent. The pH reagent may facilitate determination of a pH of the biological sample. The method may further comprise a step of combining at least three portions of the biological sample with three different reagents prior to the step of recording. For example, the method may comprise combining a third portion of the biological sample with a fixation reagent. The fixation reagent may facilitate determination of morphology of the cells contained in the biological sample. In the biological sample is semen, the fixation reagent may also facilitate determination of spermatozoa DNA fragmentation. Thus, the invention provides a method for determination of at least three parameters of cells contained in a biological sample, wherein the method comprises the steps of:

(a) combining (i) a first portion of the biological sample with a first reagent and (ii) a second portion of the biological sample with a second reagent;

(b) recording with an image sensor (i) a first video of the first portion of the biological sample; (ii) a second video of the second portion of the biological sample; and (iii) a third video of a third portion of the biological sample; and

(c) analysing the first, second, and third recorded videos of the biological sample to determine at least three parameters of the cells in the biological sample, wherein the at least three parameters are selected from morphology, vitality, pH, total count, concentration and motility.

Preferably, the image sensor is a camera. Preferably, the method is for point-of-care determination of at least three parameters of cells. Thus, the invention provides a method for point-of-care determination of at least three parameters of cells contained in a biological sample, wherein the method comprises the steps of:

(a) combining (i) a first portion of the biological sample with a vitality reagent; and (ii) a second portion of the biological sample with a pH reagent;

(b) recording with a camera (i) a first video of the first portion of the biological sample; (ii) a second video of the second portion of the biological sample; and (iii) a third video of a third portion of the biological sample; and

(c) analysing the first, second, and third recorded videos of the biological sample to determine at least three parameters of the cells in the biological sample, wherein the at least three parameters are selected from morphology, vitality, pH, total count, concentration and motility, preferably at least two of the at least three parameters are vitality and pH.

The method may comprise, before the step of combining the portion of the biological sample with a reagent, a step of providing a first, second and third portion of the biological sample. The step of providing a first, second and third portion of the biological sample may comprise obtaining a sample from a subject. The sample obtained from the subject may then be divided to form a first, second and third portion of the biological sample.

The term "video" as used herein is understood to encompass a sequence or series of image frames. For example, a sequence of serialized image frames. Thus, the expression "recording a video" may be substituted with the expression "taking a series of images" (or "taking a series of image frames").

Preferably, the biological sample is a liquid biological sample. That is to say that before the sample is combined with a reagent, the sample is in the form of a liquid. The method may further comprise a step of liquefaction of the biological sample before the step of combining the portion(s) of the biological sample with the reagent(s). The biological sample is also preferably a liquid biological sample during a step of recording the videos of the biological sample. For example, before the biological sample is combined with a reagent that is used to facilitate determination of the parameter(s) of the cell, the sample may be combined with a liquefaction reagent. Alternatively or additionally, once the biological sample is obtained from a subject, the sample may be allowed to liquefy prior to the step of combination with a reagent. This, for example, may comprise leaving the sample in a container for at least 10 or at least 15 minutes to undergo liquefaction. The liquid biological sample is particularly suitable for use in the present methods, devices, systems and kits because the methods, devices, systems and kits operate on the basis of applying and analysing biological samples that are in the liquid form. The term "liquid" is intended to encompass samples comprising cells that are substantially surrounded by a liquid medium (e.g. a buffer or a biological solution). Thus, the cells preferably are not dried out during analysis.

The methods, devices, systems and kits described herein are suitable for determining at least three parameters of the cells contained in the biological sample. Preferably the methods, devices, systems, and kits determine at least four or at least five parameters of the cells contained in the biological sample. More preferably still, the methods, devices, systems and kits determine at least 6 parameters of the cells contained in the biological sample. The parameters may be selected from morphology, vitality, pH, total count, concentration and motility. If the biological sample is semen, a further parameter the methods, devices, systems and kits may determine is spermatozoa DNA fragmentation. The methods described herein are also suitable for determination of the volume of the biological sample.

The at least three parameters of the cells may comprise morphology, pH and vitality. The vitality may be determined from the first video (i.e. the video of the biological sample which was combined with the vitality reagent), pH may be determined from the second video (i.e. the video of the biological sample which was combined with the pH reagent), and morphology may be determined from the third video (i.e. the video of the biological sample which was not combined with any reagent or was combined with a fixation reagent).

The at least three parameters of the cells may comprise morphology, motility and vitality.

The vitality may be determined from the first video (i.e. the video of the biological sample which was combined with the vitality reagent), and morphology and motility may be determined from the third video (i.e. the video of the biological sample which was not combined with any reagent).

If the method, devices, systems and kits are used to determine at least six parameters, the at least six parameters may be morphology, vitality, pH, total count, concentration and motility. The vitality may be determined from the first video (i.e. the video of the biological sample which was combined with the vitality reagent), pH may be determined from the second video (i.e. the video of the biological sample which was combined with the pH reagent), and morphology, total count, concentration and motility may be determined from the third video (i.e. the video of the biological sample which was not combined with any reagent).

The method may further comprise determination of the same parameter from multiple videos. For example, morphology, total count, concentration and motility may be determined from the first video, the second video and/or the third video. Thus, the method may include a step of determination of morphology from the first video, the second video and the third video. The methods may include a step of determination of motility from the first video, the second video and the third video. Determination of parameters from multiple videos (which might include a biological sample combined with a different reagent) provides an advantage of reducing an error associated with determination of the parameters from a single video. In the methods, devices, systems and kits described herein, vitality, morphology, total count, concentration and motility may be determined from the first video (i.e. the video of the biological sample which was combined with the vitality reagent), pH, morphology, total count, concentration and motility are determined from the second video (i.e. the video of the biological sample which was combined with the pH reagent), and morphology, total count, concentration and motility are determined from the third video (i.e. the video of the biological sample which was not combined with any reagent).

The expression "the video of the biological sample which was not combined with any reagent" is intended to encompass any reagent that is a dye or staining reagent, i.e. a reagent that can affect the colour of the tested biological sample. Reagents, such as a liquefaction reagent, are not intended to be encompassed. Thus, preferably, morphology, total count, concentration and motility are determined from a portion of the biological sample that does not comprise a dye or a stain.

In the methods, devices, kits and systems described herein, the determination of morphology, total count, concentration and/or motility may comprise averaging the determination of these parameters from the first, second and third recorded videos.

The at least three parameters of the cells in the biological sample may be determined by averaging the determination of these parameters from the first, second and third recorded videos.

The method may further comprise a step of recording with a camera a fourth video of a fourth portion of the biological sample. Preferably, the fourth portion of the biological sample is not combined with any reagent (other than, optionally, a liquefaction reagent). The fourth video may function as a control or a reference video, or may be used to (further) reduce an error in determination of parameters of the cells by providing a further recording of the cells contained in the biological sample for analysis.

The step of analysing the first, second and third recorded videos may be performed on an electronic apparatus, such as a smartphone, a tablet, or a computer, or by a software application. The step of analysing the first, second and third recorded videos may be performed on a software application of the electronic apparatus. The step of analysing the first, second and third recorded videos may be performed in a cloud-based system. The step of analysing the first, second and third recorded videos may be performed of the device as described herein.

Different biological samples may be tested using the methods, devices, systems and kits described herein. The biological sample may be semen, blood, saliva, urine, bile, bone marrow aspirate, breast milk, cerebral spinal fluid, plasma, serum, sputum, stool, oral swab, nasal swab, vaginal fluid, or synovial fluid. The biological sample may comprise a sample taken from water or any other environment (e.g. soil, air, or surface swab). Preferably, the biological sample is semen. The cells contained in the biological sample may be sperm cells, ova, red blood cells, white blood cells, platelets, neuroglial cells, muscle cells, stem cells, nerve cells, cartilage cells, bone cells, endothelial cells, or fat cells. The cells may also be pathogens, such as bacteria, single cell organisms, fungi or viruses. The biological sample may be processed prior to application of the present methods or use of the present devices, systems or kits. For example, the cells may be cultured for a period of time to allow growth or stabilisation in an artificial medium. The processing of the biological sample may comprise (i) immersion in agarose microgel, (ii) treatment with acid denaturation and lysis solution and (iii) dehydration. The dehydrated sample may be then stained with a reagent for testing of DNA fragmentation (e.g. spermatozoa DNA fragmentation).

The first, second and third portions of the biological sample may have the same or different volumes. Preferably, the portions of the biological sample have the same volume. If a fourth portion of the biological sample is collected or used, this portion may also have the same volume as the first, second and third portions. The first, second and third (and optionally fourth) portions of the biological sample may have a volume of at least 1μL, at least 2μL, at least 3μL, at least 4μL, at least 5μL, at least 10μL, at least 15μL, at least 25μL, at least 50μL, at least 100μL, at least 200μL, at least 300μL, at least 400μL, at least 500μL, at least 600μL, at least 700μL, at least 800μL, at least 900μL, at least 1mL, at least 1.1mL, at least 1.2mL, at least 1.3 mL, at least 1.4mL or at least 1.5mL

The methods described herein may utilize different reagents for combining with the biological sample to facilitate determination of the parameter(s) of the cells. The reagent may be a vitality reagent, a pH reagent, a liquefaction reagent, a DNA integrity reagent, a motility buffer, a fluorescent reagent, an acrosome reaction reagent, a fixation reagent, and/or a nutrient buffer. Preferably, a different reagent is combined with a different portion of the biological sample. For example, a vitality reagent may be combined with the first portion of the biological sample and a pH reagent may be combined with the second portion of the biological sample. Preferably, at least one portion of the biological sample does not include a reagent (other than liquefaction reagent or a non-staining buffer, if used). That is to say that, preferably, at least one portion of the biological sample does not include a dye or a stain.

The vitality reagent may be an Eosin-Nigrosin reagent. The pH reagent may be Bromothymol Blue. The liquefaction reagent may be trypsin or alpha-chymotrypsin. The DNA integrity reagent may comprise hyaluronic acid. The motility buffer may comprise sodium bicarbonate, HEPES or an antioxidant (e.g. pentoxifylline). The fluorescent reagent may be DAPI (4',6-diamidino-2-phenylindole) or a fluorescent Ca2+ indicator dye (e.g. Fura-2 or Fluo-4). The acrosome reaction reagent may comprise calcium ionophore. The fixation reagent may comprise formaldehyde or ethanol. The nutrient buffer may comprise bovine serum albumin.

The methods, devices, systems and kits described herein envisage the use of a pH meter instead of a pH reagent for determination of a pH of the biological sample.

In the methods described herein the step of combining a first portion of the biological sample with a first reagent, a second portion of the biological sample with a second reagent, and, optionally, a third portion of the biological sample with a third reagent may be performed in any order. Typically, the order will depend on the recommended length of the incubation of the reagent with the biological sample.

In the methods described herein, the steps of recording with an image sensor (e.g. a camera) of a first video of the first portion of the biological sample; a second video of the second portion of the biological sample; and a third video of the third portion of the biological sample may be performed in any order. The methods described herein also envisage repetition of the step of recording any one (or all) videos of the biological sample. For example, if the recorded video was of inadequate quality, the user of the methods, devices, systems or kits may be asked to repeat the step of recording.

The methods described herein may further comprises a step of collecting the biological sample before a step of combining the biological sample with one or more reagents.

The method may further comprise a step of measuring the volume of the biological sample. For example, the volume may be measured once the biological sample has been collected and before combination with the reagent(s). The volume may be measured by providing a measuring container. In the measuring container, the volume may be determined by checking the metric sale on the side of the container.

The methods described herein may utilize a solid support for application of the first, second and third portions of the biological sample. The solid support may be the medium where the first, second and third portion of the biological sample are combined with the reagent(s). Alternatively, the first, second and third portion of the biological sample are combined with the reagent(s) prior to the application of the samples to the solid support.

The method may further comprise a step of applying (i) the first portion of the biological sample to a first location on a solid support, (ii) the second portion of the biological sample to a second location on a solid support, and (iii) the third portion of the biological sample to a third location on a solid support. This step may be performed before or after the step of combination of the portions of the biological sample with the reagent(s). More than one solid support may be used. The method may comprise a step of applying (i) the first portion of the biological sample to a first solid support, (ii) the second portion of the biological sample to a second solid support, and (iii) the third portion of the biological sample to a third solid support.

The solid support may be a slide. Preferably, the solid support is a glass slide. The solid support may comprise at least three cell chambers. If the solid support comprises at least three chambers, the first portion of the biological sample may be applied to a first cell chamber of the solid support (e.g. slide), the second portion of the biological sample may be applied to a second cell chamber of the solid support (e.g. slide), and the third portion of the biological sample may be applied to a third cell chamber of the solid support (e.g. slide). The solid support may comprise four chambers. If four chambers are present, the fourth chamber may be left empty or may comprise a fourth potion of the biological sample. Preferably, the fourth portion of the biological sample does not comprise a reagent (other than a liquefying regent, or a non-staining buffer., if used).

The cell chamber may have a depth of between about 5 $\mu$m and about 20 $\mu$m. In some embodiments, the cell chamber has a total volume of between about 0.01 $\mu$l and about 100 $\mu$l. In some embodiments, the cell chamber is optically transparent over the wavelength range of about 400 nm to about 2,500 nm.

In the methods, systems, devices and kits described herein, each of the first, second and third videos may be at least 1 second, at least 2 seconds, at least 3 seconds, at least 4 seconds, at least 5 seconds, at least 6 seconds, at least 7 seconds, at least 8 seconds, at least 9 seconds, at least 10 seconds, at least 11 seconds, at least 12 seconds, at least 13 seconds, at least 14 seconds, at least 15 seconds, at least 16 seconds, at least 17 seconds, at least 18 seconds, at least 19 seconds, 20 seconds, at least 25 seconds, at least 30 seconds, at least 35 seconds, at least 40 seconds, at least 45 seconds, or at least 50 seconds in length. Preferably, each of the first, second and third videos is at least 5 or at least 10 seconds in length. A length of the recorded video over 2, preferably 10, seconds allows to collect enough data to perform a comprehensive analysis of motility of the cells contained in the biological sample. Specifically, it allows to correct for any errors associated with collecting data from a shorter video.

The methods, devices, systems and kits may further comprise a step of outputting the at least three, preferably six, parameters of the cells in the biological sample, after the step of analysing the first, second, and third recorded videos.

The step of outputting the at least three, preferably six, parameters of the cells in the biological sample, may be performed on an electronic apparatus, such as a smartphone, a tablet or a computer. The step of outputting the at least three, preferably six, parameters of the cells in the biological sample, may be performed in a software application on an electronic apparatus.

The step of recording with an image sensor (e.g. a camera) (i) a first video of a first portion of the biological sample; (ii) a second video of a second portion of the biological sample; and (iii) a third video of a third portion of the biological sample may be performed in a device (e.g. a point-of-care device) as described herein. The device may comprise:

(a) a port configured to receive a solid support containing a biological sample;
(b) a light source configured to direct a light beam onto the solid support received in the port;
(c) an optical system comprising at least one lens, preferably at least two lenses, wherein the optical system is configured to magnify, preferably at least 100 times, an image of the biological sample contained in or on the solid support;
(d) an image processing system comprising (i) a camera configured to record at least one, preferably at least three, video(s) of the magnified biological sample; and (ii) an image processor configured to transmit, preferably via a Wi-Fi module, the at least one, preferably at least three, video(s) of the magnified biological sample to the electronic apparatus.

In all embodiments described herein, the method is preferably performed without the manual analysis of the at least three, preferably six, parameters of the cells in the biological sample. That is to say that the parameters of the cells are determined using methods that are independent of user's analysis. For example, the user of the present methods does not have to perform a visual examination of the recorded video (or the biological sample) to, for example, count the cells. The present invention overcomes the disadvantages of manual examination of the biological sample such as high error rate and/or lack of reproducibility.

The methods, devices, systems and kits described herein provide a further advantage of determination of the at least three, preferably six, parameters of cells contained in the biological sample in under 3 minutes. This makes the methods, devices, systems and kits described herein particularly suitable for daily examination of the biological sample.

The invention also provides a device for analysis of a biological sample, wherein the device is preferably used with an electrical apparatus, wherein the device comprises:

(a) a port configured to receive a solid support containing a biological sample;
(b) a light source configured to direct a light beam onto the solid support received in the port;
(c) an optical system comprising at least one lens, wherein the optical system is configured to magnify an image of the biological sample contained in or on the solid support;

(d) an image processing system comprising (i) an image sensor configured to record at least one video of the magnified biological sample; and (ii) an image processor configured to transmit the at least one video of the magnified biological sample to the electronic apparatus.

The device may be used to determine at least three, at least four, at least five, or preferably at least six, parameters of the cells contained in the biological sample. The device may be used for analysis of the quality of a biological sample.

Preferably, the device is a point-of-care device. That is to say that the device can deliver results without any laboratory analysis. The device preferably does not require operating by trained personnel.

The image sensor may be a camera. The image processor preferably transmits the at least one video of the magnified biological sample to the electronic apparatus via a Wi-Fi module. Alternatively, the at least one video may be transmitted to the electronic apparatus by a cable communication between the device and the apparatus.

The devices described herein may be configured to perform the methods described herein. That is to say that at least one step of the methods described herein are performed on the device of the invention.

The port may be an opening in a device which can accommodate the solid support. The port may be a platform for placing the solid support. The port may comprise means for securing the solid support. The port may comprise rails for inserting the solid support. Different dimensions and shapes of the port are envisaged. Preferably, the port has dimensions which are corresponding or greater than the dimensions of the solid support.

The port may be movable relative to the optical system, the light source, and/or the image processing system. The port may be configured so that the solid support is movable or slidable inside the device. This allows moving the solid support in the device so that the optical system and/or the image processing may magnify and/or record videos of different locations on the solid support. For example, if the first portion of the biological sample is located in a first location on a solid support and the second portion of the biological sample is located in a second location on the solid support, the user or the device (e.g. if automated) may move the solid support (or the port) so that the optical system and/or the image processing system are aligned with the desired location on the solid support. This may mean that the user is required to slide the solid support inside the device, for example, upon receipt of instructions from the device or an electronic apparatus.

The port may be configured so that the light source directs a light beam onto one of at least three locations on the solid support. The light source may be an LED-light, a light bulb, or a reflector reflecting a natural light or an electrical light source onto the biological sample. The light source may emit monochromatic or polychromatic light. The light source may direct the light beam onto one of at least three locations on the solid support. The light source may be adapted to emit light that illuminates and at least partially propagates through the biological sample so that the image sensor (e.g. camera) receives at least part of the emitted light. In some embodiments, the light source provides light in the wavelength range from about 400 nm to about 700 nm.

Preferably, the port is located between the light source and the optical system. That is to say that the light source may be located below the port to illuminate the biological sample from below. In alternative embodiments, the light source may be located on the side of the port to illuminate the biological sample on the solid support from a side. In alternative embodiments, the light source may be located above the port to illuminate the biological sample on the solid support from above (without obstructing the view of the sample for the image sensor).

The light source may be connected to a power source, such as a low voltage wire for connecting to an outside source of energy, a battery, a power adapter, or a charger.

Preferably, the optical system is arranged between the port and the image processing system.

The optical system may comprise two, three, four or five lenses. Preferably the optical system comprises two lenses.

The at least one lens, preferably two lenses, may magnify the image of the biological sample (at the position of the image sensor (e.g. a camera)) between 10 to 1500 times, preferably between 100 and 1000 times. The at least one lens, preferably two lenses, may magnify the image of the biological sample at least 50 or at least 100 times. In all aspects and embodiments described herein, the optical system preferably magnifies the image of the biological sample at least 100 times, preferably between 100- 400 times. The optical system may magnify the image of the biological sample between 100-1000 times. In a preferred embodiment, the optical system magnifies the image of the biological sample 100-250 times. The optical system may comprise an objective lens and an eyepiece. The objective lens may magnify the image of the biological sample between 10-40 times. The eyepiece may magnify the image of the biological sample between 10-25 times. The devices or systems described herein allow to obtain a video or an image of high resolution. The resolution may be between 10 nm to 250 nm. Preferably, the resolution is at least 200 nm. More preferably still, the resolution is at least 50 nm. The high resolution used in the devices and systems of the invention allows to provide clinically meaningful data for patients. Surprisingly, the inventors of the present invention discovered devices and systems that are particularly suitable for high quality point of care analysis of the biological sample. This was, partially, accomplished due to the use of an optical system which is capable of delivering a high magnification and high resolution of the sample image, which allows for high quality identification and tracking individual cells through different frames of the recorded video (or through different images in the series of images taken).

The lens (or lenses) of the optical system may be a ball lens, concave lens, convex lens, an aspheric lens, a piano-convex lens, a convexo-concave lens, or a GRIN lens. The lens (or lenses) of the optical system may be any lens(es) that are typically used in microscopes, for example the lens(es) may be objective lens(es) or an eyepiece. The objective lens(es) may be achromatic or chromatic microscope objective lens(es). The magnification of the lens may be at least 10 times, preferably at least 25 times. If two lenses are used, the total magnification from the optical system may be between 100-1000. For example, each of the two lenses (e.g. an objective lens and an eyepiece) may have magnification of 10 times. The optical system may comprise one or more microlens arrays (instead of or in addition to the at least one lens).

Thus, the point-of-care device may comprise:

(a) a port configured to receive a solid support containing a biological sample;
(b) a light source configured to direct a light beam onto the solid support received in the port;
(c) an optical system comprising two lenses, wherein the optical system is configured to magnify an image of the biological sample contained in or on the solid support at least 100 times;
(d) an image processing system comprising (i) a camera configured to record at least one, preferably at least three, video(s) of the magnified biological sample; and (ii) an image processor configured to transmit, preferably via a WiFi module, the at least one, preferably at least three, video(s) of the magnified biological sample to the electronic apparatus.

The lens (or lenses) may be movable using an actuator, a piezoelectric motor, a voice coil magnetic driver or mechanical displacement.

The optical system may further comprise a body tube. The body tube may be positioned between the at least one lens and the image processing system. Preferably, the body tube is positioned between two lenses.

The image sensor, preferably a camera, may be configured to record at least two, at least three or at least four videos of the magnified biological sample, preferably at least three videos of the magnified biological sample. The first of the at least three videos may be a video of a first of the at least three locations on the solid support. The second of the at least three videos may be a video of a second of the at least three locations on the solid support. The third of the at least three videos may be a video of a third of the at least three locations on the solid support.

In some embodiments, a field-of-view of the optical system is about 0.1 mm × 0.1mm, about 0.5mm × 0.5mm, or about 1mm × 1mm.

In some embodiments, the optical system further comprises at least one additional lens, mirror, dichroic reflector, prism, optical filter, optical fiber, aperture, light source, image sensor chip, or any combination thereof.

The image sensor may record a series of images or a video in high fidelity image resolution. For example, the resolution may be 640 × 480 pixels, 1280 × 720 pixels, 1920 × 1080 pixels, 3840 × 2160 pixels, or 7680 × 4320 pixels.

Instead or in addition to lens(es) used in the standard light microscopy, the device of the present invention may comprise an optical system comprising means for converting phase shifts in the light beam passing through the biological sample to brightness changes. That is to say that the device may include elements of a phase contrast microscope. The elements may include at least one condenser, a condenser annulus, an objective (i.e. a phase contrast objective), at least one phase plate (e.g. comprising a phase shift ring), and/or at least one filter ring (e.g. a gray filter ring). The phase plate may be a -90° phase plate or a +90° phase plate.

Thus, the invention provides a device for analysis of a biological sample, wherein the device is preferably used with an electrical apparatus, wherein the device comprises:

(a) a port configured to receive a solid support containing a biological sample;
(b) a light source configured to direct a light beam onto the solid support received in the port;
(c) an optical system comprising means for converting phase shifts in the light beam passing through the biological sample to brightness changes; and
(d) an image processing system comprising (i) a camera configured to record at least one video of the biological sample; and (ii) an image processor configured to transmit, preferably via a Wi-Fi module, the at least one video of the biological sample to the electronic apparatus.

The device comprising elements of the phase contrast microscope is preferably used to examine biological sample without any reagent (other than the liquidation reagent). The phase contrast elements of the device may be used to determine morphology of the cell. If the biological sample is semen, the phase contrast elements of the device may be used to determine spermatozoa DNA fragmentation.

If the biological sample is blood, the phase contrast elements of the device may be used to determine the morphology of blood cells like erythrocytes (red blood cells), leukocytes (white blood cells), and platelets. Any changes in size, shape, and intracellular structures may be detected. Internal structures like nuclei, granules in granulocytic white blood cells, or potential inclusions in red blood cells may be visualized more clearly. Similarly, the phase contrast elements of the device may be used to study the movement of live cells in a biological sample without the need for staining. The use of phase contrast elements is also particularly suitable for detection of blood parasites, such as Plasmodium. If the biological sample is a plasma or serum sample, the phase contrast elements may enhance the visibility of fibrin strands and other clotting elements.

The image processor may be configured to modify the at least one recorded video to remove or reduce neural networks inference. The image processor may be configured to modify the colour balance of the at least one recorded video. In some embodiments, the image processing system is configured to acquire bright-field, dark-field, phase contrast, or fluorescence images.

The Wi-Fi module may be connected to a power source, such a wire for connecting to an outside source of energy, a battery, a power adapter, or a charger.

The device may further comprise means for adjusting focus of the image of the biological sample. The focus may be adjustable by manual operation upon receipt of instructions from the electronic apparatus. Alternatively or additionally, the device may further comprise means for an automated adjustment of the focus configured to adjust the focus of the image of the biological sample, for example, upon receipt of instructions from the electronic apparatus and/or the image processor. The means for an automated adjustment of the focus may be an actuator, an auto-focus step motor, a piezoelectric motor, a voice coil magnetic driver or a mechanical displacement.

The means for an automated adjustment of the focus may be configured to move the port, the optical system (e.g. an eye piece or the at least one lens), and/or the image processing system (e.g. an image sensor (e.g. camera)). The solid support may be mounted on the means for an automated adjustment of the focus (e.g. an actuator). The means for an automated adjustment of the focus (e.g. an actuator) may move the solid support along the x, y, z axis to reach an optimal focus. The optimal focus may be outputted on the camera. The means for an automated adjustment of the focus may be connected to a power source, such as a wire for connecting to an outside source of energy, a battery, a power adapter, or a charger. Preferably, the means for an automated adjustment of the focus is configured to adjust the focus of the image of the biological sample upon receipt of instructions from the electronic apparatus (or the software application of the electronic apparatus) and/or the Wi-Fi module. The electronic apparatus (or the software application of the electronic apparatus) may perform an algorithm to detect the sharpness of the edges of cells of the recorded video. If the sharpness is not within the acceptable range, the electronic apparatus (or the software application of the electronic apparatus) may send a command to the device so that the focus of the recorded image is adjusted. This step may be performed multiple times until the sharpness of the cell edges is within the acceptable range.

The device may be less than 50cm, less than 45 cm, less than 40cm, less than 35 cm, less than 30 cm, less than 25 cm, less than 20 cm, or less than 15 cm in height.

The device may further comprise a housing configured to hold the port, the light source, the optical system, and the image processing system. The housing may comprise an opening for inserting the solid support into the port. Preferably, the housing allows for movement of the solid support in the device.

The device may further comprise a pH meter (e.g. for embodiments in which a pH reagent is not used), an environment sensor (e.g. a heat sensor or a humidity sensor), and/or a biological sample sensor.

The biological sample is preferably semen.

The device described herein may be for point-of-care analysis of the quality of a biological sample, wherein the device is preferably used with an electrical apparatus, the device comprising:

(a) a port configured to receive a solid support containing a biological sample;
(b) a light source configured to direct a light beam onto the solid support received in the port;
(c) an optical system comprising at least one lens, wherein the optical system is configured to magnify an image of the biological sample contained in or on the solid support;
(d) an image processing system comprising (i) a camera configured to record at least one video of the magnified biological sample; and (ii) an image processor configured to transmit, preferably via a Wi-Fi module, the at least one video of the magnified biological sample to the electronic apparatus.

The device described herein may be for point-of-care determination of at least three parameters of cells contained in a biological sample, wherein the device is preferably used with an electrical apparatus, the device comprising:

(a) a port configured to receive a solid support containing a biological sample;
(b) a light source configured to direct a light beam onto the solid support received in the port;
(c) an optical system comprising at least one lens, wherein the optical system is configured to magnify an image of the biological sample contained in or on the solid support;
(d) an image processing system comprising (i) a camera configured to record at least one video of the magnified biological sample; and (ii) an image processor configured to transmit, preferably via a Wi-Fi module, the at least one video of the magnified biological sample to the electronic apparatus.

The invention also provides a system for analysis of at least one parameter of a cell contained in a biological sample, the system comprising:

a device comprising:

an image sensor configured to record at least three videos of the biological sample; and
at least one processor communicatively connected to the image sensor, the at least one processor being configured to:

a) receive the at least three recorded videos of the biological sample captured by the image sensor;
b) transmit, preferably via a Wi-Fi module, the at least three recorded videos of the biological sample to an electronic apparatus;

the electronic apparatus configured to receive the at least three recorded videos of the biological sample, and to:

a) return distribution of motility of a cell in the biological sample, wherein the distribution of motility is obtained from the average velocity of the cell; and/or
b) return morphology of a cell, wherein morphology of the cell categorizes the cell as normal (i.e. healthy) or abnormal (i.e. unhealthy);

and wherein the at least one parameter is selected from morphology and motility.

Preferably, the biological sample is semen. Preferably, the system is used in point-of-care analysis of at least one, preferably at least three, parameter(s) of a cell contained in a biological sample.

The distribution of motility may be obtained by the following steps:

i. identify a cell in a first frame of the first of the at least three recorded videos;
ii. identify the cell in a second frame of the first of the at least three recorded videos;
iii. identify the cell in a third frame of the first of the at least three recorded videos;
iv. obtain (i) an average velocity of the cell based on the first, second and third frames of the first of the at least three recorded videos; and
v. return distribution of motility of the cell in the biological sample, wherein the distribution of motility is obtained from the average velocity of the cell.

Preferably, the cell is identified in at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, or at least 50 frames of the first of the at least three recorded videos. Preferably, the average velocity of the cell is obtained based on at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, or at least 50 frames of the first of the at least three recorded videos.

The first and second frames may be separated by an interval of between 1/24 to 1/240 second. The second and third frames may be separated by an interval of 1/24 to 1/240 second. If more frames (e.g. 50 frames) are used, the interval between each two consecutive frames is preferably 1/50 second. The cell may be tracked between the first and second frames. The cell may be tracked between the second and third frames. If more frames (e.g. 50 frames) are used, the cell may be tracked between each two consecutive frames. The average velocity may be obtained based on tracking the cell between the frames of the recorded video. The average velocity may be obtained based on velocities of the cell obtained from each two consecutive frames in at least 24, at least 30, at least 50, at least 60, at least 120, or at least 240 frames of the first of the at least three recorded videos. The video may record 24, 25, 30, 50, 60, 120, or 240 frames per second.

The average velocity may be obtained between the first and last frame of the recorded video. For example, if the recorded video has 50 frames, the average velocity may be obtained from each individual frames between the first frame and the 50th frame of the recorded video.

The average velocity may be determined using the equation:

$$v_i = \frac{1}{T-1s} \sum_{t=0}^{T-1s} \frac{\Delta x_i(t)}{1s}$$

where $T$ is the length of the recorded video;

for each cell i and each time t, the displacement $\Delta x_i(t)$ is computed as the Euclidean distance between its positions in moments t and $(t+ 1s)$.

The morphology may be obtained by the following steps:

i. identify a cell in a first frame of the first of the at least three recorded videos to obtain an image of the cell;
ii. apply a machine-learning classification model to the image of the cell;
iii. return morphology of a cell, wherein morphology of the cell categorizes the cell as normal (i.e. healthy) or abnormal (i.e. unhealthy).

The morphology may be obtained by the following steps:

i. identify a cell in a first frame of the first of the at least three recorded videos to obtain an image of the cell;
ii. apply a machine-learning classification model to the image of the cell;
iii. determine at least one category of cell features which the cell exhibits based on the image of the cell from the first of the at least three recorded videos,
iv. return morphology of a cell, wherein morphology of the cell categorizes the cell as normal (i.e. healthy) or abnormal (i.e. unhealthy).

The determination of the morphology of a cell may further return the category of the cell features of the cell and the likelihood of the cell features belonging to the determined category. The cell features may be abnormal cell features. The cell features may be associate with a disease state of the cell. That is to say that the determination of the morphology may return a category of morphology defects.

Preferably, a plurality of cells is identified. In the context of motility, plurality of cells may be identified in the first, second and third frames of the first of the at least three recorded videos, the average velocity may be obtained for each cell in the plurality of cells, and the distribution of motility may be obtained from the average velocities of each cell in the plurality of cells.

In a preferred embodiment, the system is for point-of-care analysis of at least three parameters of a plurality of cells contained in a biological sample, wherein the biological sample is semen, the system comprising:

a. a device comprising:

(i) a camera configured to record at least three videos of the biological sample; and
(ii) at least one processor communicatively connected to the camera, the at least one processor being configured to:

a) receive the at least three recorded videos of the biological sample captured by the camera;
b) transmit, preferably via a Wi-Fi module, the at least three recorded videos of the biological sample to an electronic apparatus;

b. the electronic apparatus configured to receive the at least three recorded videos of the biological sample, and to:

A.

i. identify a cell from a plurality of cells in a first frame of the first of the at least three recorded videos; identify the cell from a plurality of cells in a second frame of the first of the at least three recorded videos; identify the cell from a plurality of cells in a third frame of the first of the at least three recorded videos;
ii. repeat step i. for a second cell from a plurality of cells;

iii. obtain average velocities of the first and second cells based on the first, second and third frames of the first of the at least three recorded videos; and
iv. return distribution of motility of the first and second cells in the biological sample, wherein the distribution of motility is obtained from the average velocities of the first and second cells; and

B.

i. identify a first cell in the first frame of the first of the at least three recorded videos to obtain an image of the first cell; identify a second cell in the first frame of the first of the at least three recorded videos to obtain an image of the second cell;
ii. apply a machine-learning classification model to the images of the cells;
iii. determine at least one category of cell features which the first cell exhibits based on the image of the first cell from the first of the at least three recorded videos,
iv. repeat step iii. for the second cell;
iv. return the categories of the cell features of the cells in the biological sample;

wherein the at least two parameters are morphology and motility.

The cell from the plurality of cells may be identified in *n* number of frames. The n number may be any number between 10 to 600, preferably between 24 to 240. The cell may be tracked between the first frame and the $n^{th}$ frame. The average velocity may be determined based on the distance travelled by the cell between the first cell and the $n^{th}$ cell.

The step of identifying a first cell in the first frame of the first of the at least three recorded videos to obtain an image of the first cell and identifying a second cell in the first frame of the first of the at least three recorded videos to obtain an image of the second cell may be performed by a machine-learning object detection model.

The system may further determine the pH from a first frame of the second of the at least three recorded videos. The system may further determine the vitality from a first frame of the third of the at least three recorded videos. The system may further determine the total cell count and/or concentration from a first frame of the first of the at least three recorded videos.

The determination of vitality may be performed by the following steps:

i. identify a cell in a first frame of the first of the at least three recorded videos to obtain an image of the cell, wherein the first of the at least three recorded videos is obtained from a portion of a biological sample which was combined with a vitality reagent;
ii. apply a machine-learning classification model to the image of the cell; and
iii. return vitality of a cell, wherein vitality of the cell categorizes the cell as live or dead.

The determination of a pH may be performed by the following steps:

i. apply a machine-learning classification model to an image of the biological sample; and
ii. return a pH of the biological sample.

The machine-learning classification model may determine the pH of the biological sample based on the colour of the image of the biological sample.

The determination of concentration may be performed by the following steps:

i. identify all cells of interest (e.g. sperm cells) in a first frame of the first of the at least three recorded videos;
ii. return concentration of the biological sample, wherein the concentration is obtained by dividing the number of identified cells of interest by the volume of the sample in the first frame.

The volume of the sample in the first frame may be computed as the thickness of the sample layer (fixed by the known height of the cell chamber) multiplied by the area visible to the image sensor (e.g. camera).

The determination of the total count may comprise multiplying the cell concentration by the total volume of the collected biological sample.

The determination of spermatozoa DNA fragmentation may comprise the steps:

i. identify a cell in a first frame of the first of the at least three recorded videos to obtain an image of the cell, wherein the first of the at least three recorded videos is obtained from a portion of a biological sample which was combined with a reagent for staining the cell;

ii. apply a machine-learning classification model to the image of the cell; and

iii. return a spermatozoa DNA fragmentation of a cell, wherein the spermatozoa DNA fragmentation of the cell categorizes the cell as showing DNA fragmentation or not showing DNA fragmentation.

The machine-learning classification model may comprise a neural network classifier. The machine-learning classification model may be configured to classify the cells as normal or abnormal. The abnormal cells may be classified as (i) having head defect(s), (ii) having neck and midpiece defect(s), (iii) having tail defect(s), or (iv) having cytoplasmic droplet(s). The machine-learning classification model may be configured to output acrosome coverage, tetrazoospermia index, deformity index, and/or multiple anomalies index.

The distribution of motility may comprise rapidly progressive, slowly progressive, non-progressive, and immotile cells.

Preferably, the system may analyse at least two parameters of a cell. The at least two parameters of a cell may be motility and morphology. The system may further analyse pH, total count, concentration, or vitality. If the biological sample is a semen sample, the system may further analyse spermatozoa DNA fragmentation.

The motility, morphology, concentration and/or total count may be determined from each one of the first, second and third recorded videos. The final result for each parameter may reflect the average result obtained from the first, second and third recorded videos. If more videos are recorded, the final result for each parameter may reflect the average result obtained from all recorded videos.

In some embodiments, the processing of image or video data comprises applying algorithms for object detection and/or tracking. In some embodiments, the processing of image or video data comprises applying a contrast adjustment algorithm, a noise reduction algorithm, a flat-field or vignetting correction algorithm, an optical distortion correction algorithm, an optical aberration correction algorithm, a data compression algorithm, an image stabilization algorithm, or any combination thereof to the series of one or more image(s) or to the frames of the recorded video.

In some embodiments, video or image acquisition by the image sensor is controlled by a software application running on the electronic apparatus (e.g. a smartphone). In some embodiments, the software application performs further processing of the image data that comprises performing an object identification algorithm, an object tracking algorithm, an object categorization algorithm, a pH determination algorithm, a vitality determination algorithm. In some embodiments, the further processing of the image or video data provides a test result for total cell count, concentration, distribution of cell motility, cell vitality, cell morphology (e.g. categorization of normal or abnormal cells, or assignment of each cell to different categories of abnormal cells).

In some embodiments, the electronic apparatus comprises a software application that is configured to upload image or video data or a test result to a cloud-based database. Some, most or all of the analysis may be performed in the cloud using a cloud-based image or video processing algorithm, on the electronic apparatus, or on the device as described herein. Thus, some, most of all of the diagnostic or medical insights on the recorded video may rely on inference of AI model in the methods, devices, systems and kits described herein. The image or video processing algorithm may comprise the use of a machine learning algorithm. The machine learning algorithm may comprise a supervised machine learning algorithm. The supervised machine learning algorithm may comprise an artificial neural network, a decision tree, a logistical model tree, a Random Forest, a support vector machine, or any combination thereof. The machine learning algorithm may comprise an unsupervised machine learning algorithm. The unsupervised machine learning algorithm may comprise an artificial neural network, an association rule learning algorithm, a hierarchical clustering algorithm, a cluster analysis algorithm, a matrix factorization approach, a dimensionality reduction approach, or any combination thereof.

The image sensor is preferably a camera. The device in the system preferably comprises an optical system comprising at least two lenses as described herein. The optical system may magnify the image of the biological sample between 100 to 1000 times.

The electronic apparatus may be a smartphone, a tablet, or a computer. The electronic apparatus may comprise a software application for displaying the results of the analysis and/or for providing instructions to the user (e.g. to adjust focus or to move the solid support to the next tested location).

The system may comprise the device as described herein.

The invention also provides a method for testing the quality of a biological sample, preferably semen, the method comprising the steps:

(a) recording a video of the biological sample with a camera;

(b) identifying a plurality of cells in the biological sample based on the recorded video;

(c) determining a location of a first cell of the plurality of biological cells in a first frame of the recorded video; determining a location of the first cell of the plurality of biological cells in a second frame of the recorded video; and determining a location of the first cell of the plurality of biological cells in a third frame of the recorded video;

(d) obtaining an average velocity of the first cell based on the location of the first cell in the first, second and third frame of the recorded video.

Preferably, the location of the first cell in the biological sample is determined in at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, or at least 50 frames. Preferably, the average velocity of the first cell is obtained based on the location of the first cell in at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 44, at least 45, at least 46, at least 47, at least 48, at least 49, or at least 50 frames of the recorded video.

The invention provides a kit for analysis of the biological sample, the kit comprising:

(a) the device as described herein; and
(b) a solid support.

The kit may further comprise one or more reagents for combining with the biological sample. Alternatively, the one or more reagents may be deposited on the solid support. The kit may comprise at least two or at least three solid supports.

The invention provides a test kit for use with the device as described herein or the system as described herein, the test kit comprising:

(a) a solid support; and
(b) one or more reagents for combining with the biological sample.

The invention also provides a test kit for use with the device as described herein or the system as described herein, the test kit comprising a solid support, which has one or more reagents for combining with the biological sample distributed on or in the solid support.

The kit or test kit may further comprise at least one container for storing the collected biological sample. The container may comprise means for measuring volume of the biological sample.

The kit or test kit may further comprise a cable for connecting the device to the power source.

The kit or test kit may further comprise at least one pipette for transferring the biological sample to the solid support. The at least one pipette may be an exact volume pipette.

The kit or test kit may further comprise at least one container for the one or more reagents. If more than one reagent are used, the kit may comprise more than one container.

The kit or test kit may further comprise instructions for use of the kit in the methods described herein. The instructions may be displayed on the electronic apparatus (e.g. a smartphone), or in the software application on the electronic apparatus.

The invention provides a use of the system as described herein, the kit as described herein, or the device as described herein for testing the quality of a biological sample, preferably semen.

The invention provides a use of the system as described herein, the kit as described herein, or the device as described herein for diagnosing a disease associated with or resulting in male infertility.

The invention provides a use of the system as described herein, the kit as described herein, or the device as described herein for assessing male fertility.

The invention provides a use of the system as described herein, the kit as described herein, or the device as described herein for testing a biological sample, preferably semen, prior to in vitro fertilization (IVF).

The invention provides a use of the system as described herein, the kit as described herein, or the device as described herein for testing a biological sample, preferably semen, after vasectomy or reverse vasectomy.

The invention provides a use of the system as described herein, the kit as described herein, or the device as described herein in veterinary diagnostics. The invention provides a use of the system as described herein, the kit as described herein, or the device as described herein in assessing the fertility status of an animal. For example, the devices, kits and systems of the present invention may be used in livestock breeding diagnostics, e.g. during breeding soundness examination.

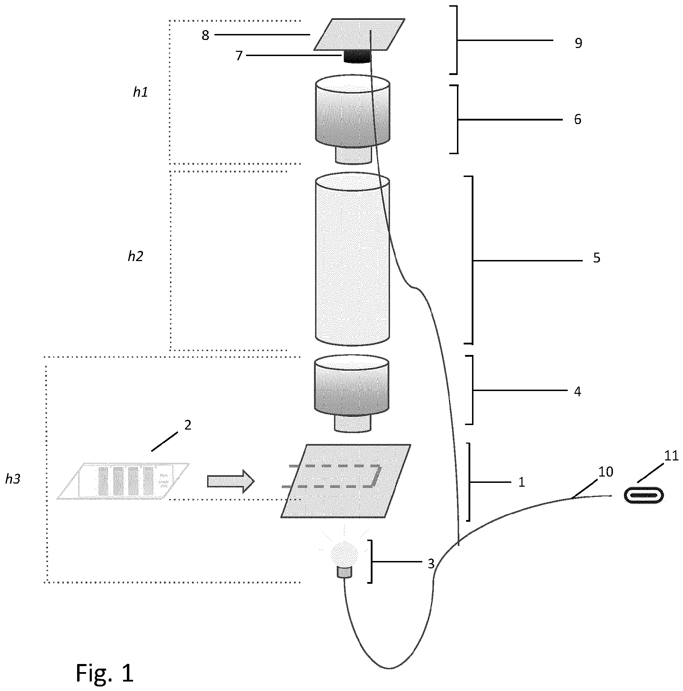

Exemplary embodiments of the invention are described with reference to accompanying figures. All features of the described devices are applicable to the described methods, systems and kits. Devices depicted in some of the figures may be provided in various configurations. In a preferred embodiment, as shown in Fig. 1, the device comprises a port 1, which is a platform, for receiving a four-chamber slide 2 containing a biological sample (e.g. semen). Once inserted in the device, the first chamber of the slide 2 is illuminated with a light source 3. The light source 3 is powerful enough so that at least a part of the light beam propagates through the sample contained in the first chamber and travels through a first magnifying lens 4, a body tube 5 and a second magnifying lens 6 and is received by the camera 7. The first magnifying lens 4, the body tube 5 and the second magnifying lens 6 form an optical system. The optical system magnifies the sample by a factor of at least 100. The resolution of the optical system is about 200 nm in the present embodiment. The camera 7 and an image processor 8 (with a Wi-Fi module in this embodiment) form an image processing system 9, which transmits the images or videos of the sample to the electronic apparatus (not shown) for analysis. The light source 3 and the image processor (including a Wi-Fi module) 8 are connected to a power source 11 with a low volage wire or cable 10. The power source 11 in this embodiment is USB Type-C port. In this embodiment, the device has h1 of 82 mm, h2 of 160 mm and h3 of 78mm; however embodiments in which the hight is much smaller are also within the scope of the invention. In operation, the camera 7 records a first video of the first chamber of the slide 2, the user is then asked to move the slide in the device so that a second video of the second chamber of the slide 2 is recorded. Once the second video is recorded, the user is asked to move the slide in the device so that a third video of the third chamber of the slide 2 is recorded. In this embodiment, the user may receive instructions from a software application of an electronic apparatus (e.g. a smartphone). Optionally, before each video is recorded, the user may be asked to perform manual adjustment of the focus. In this embodiment, the first, second and third recorded videos may be sent to the cloud-based system for analysis.

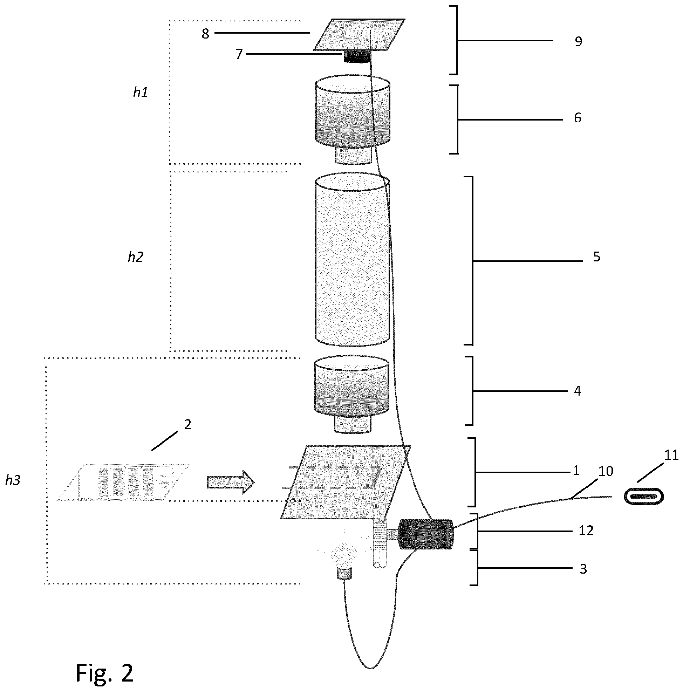

In a second preferred embodiment, illustrated in Fig. 2, the device comprises the features of the device of Fig. 1 and it additionally comprises an auto-focus step motor 12 attached to the platform 1 for moving the platform 1 to adjust the focus upon receipt of instructions from either the image processing system 9 or from the electronic apparatus directly (not shown). Thus, in this embodiment, the user does not manually adjust the focus. Although in this embodiment the auto-focus step motor 12 is attached to the platform 1, embodiments in which the auto-focus step motor 12 is attached to any elements of the optical system or the camera 7 are also within the scope of the invention.

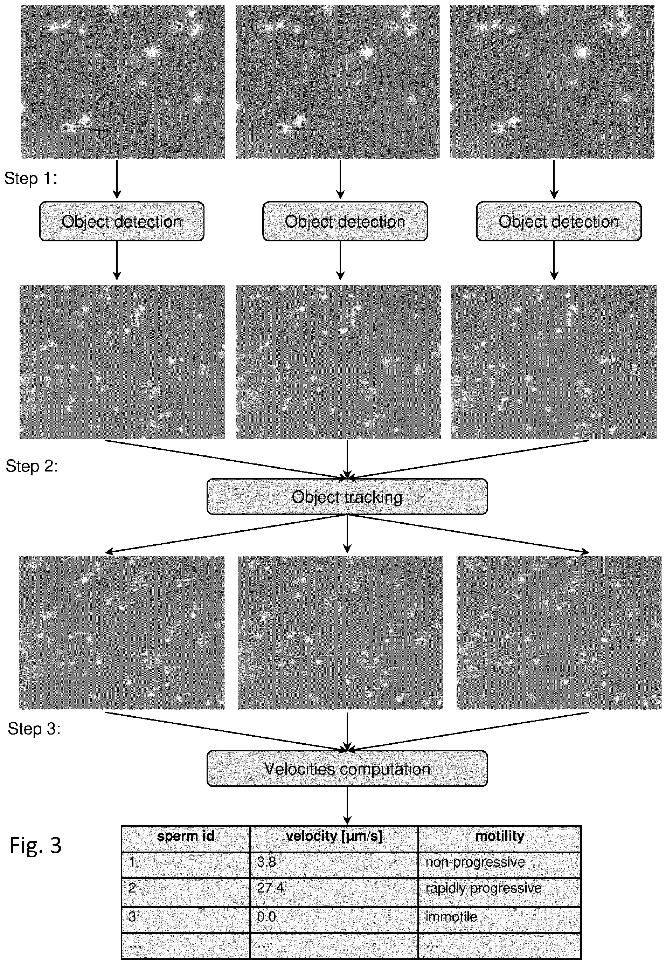

Fig. 3 illustrates exemplary workflow of the steps taken in the determination of average velocities of the cells (here sperm cells) in the biological sample. Once the images or videos of the biological sample (here semen) are taken using the devices or systems described herein, the devices or systems perform an object detection algorithm in step 1 to identify each cell in the image or the frame of a video. In step 2, each cell is tracked in multiple (preferably all) images of the series of images or in multiple (preferably all) frames of the video. In step 3, for each cell, the velocities of the cell are determined from each image in the series of images or between each frame of the video to obtain multiple velocities for each cell. For each cell, an average velocity is obtained from the determined multiple velocities. Each cell is assigned to one of four categories ((i) immotile if the average velocity is 0 $\mu$m/s, (ii) non-progressive if the average velocity is < 5 $\mu$m/s, (iii) slowly progressive if the average velocity is $\geq$ 5 $\mu$m/s and < 25 $\mu$m/s, and (iv) rapidly progressive if the average velocity is $\geq$ 25 $\mu$m/s)). The method outputs a distribution of the four categories of cells in the biological sample.

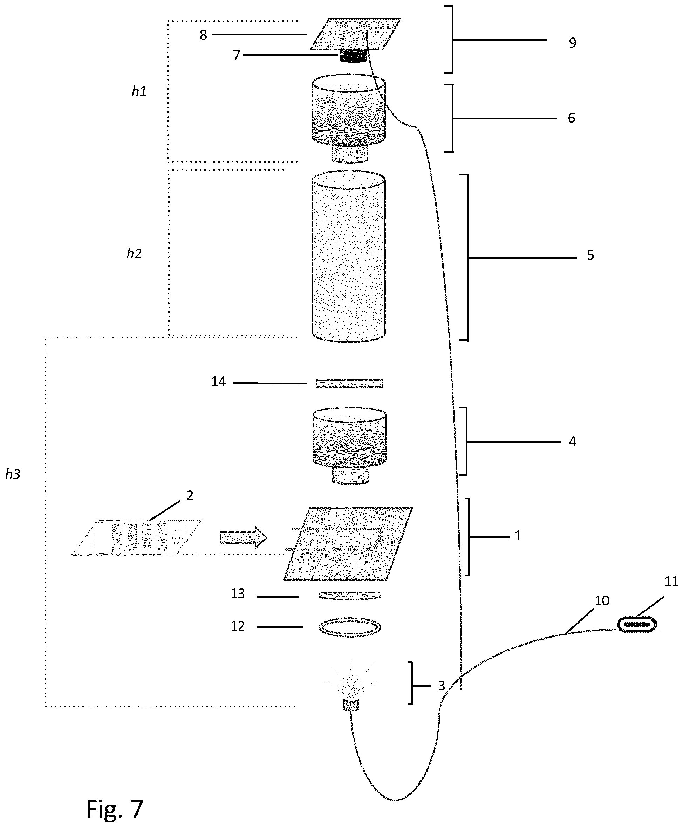

In an embodiment, illustrated in Fig. 7, the device comprises the features of the device of Fig. 1 and it additionally comprises a condenser annulus 12, a condenser 13, and a phase plate 14.

The foregoing detailed description has been provided by way of explanation and illustration, and is not intended to limit the scope of the appended claims. Many variations in the presently preferred embodiments illustrated herein will be apparent to one of ordinary skill in the art, and remain within the scope of the appended claims and their equivalents.

## EXAMPLES

The following non-limiting examples further illustrate the present invention.

**Example 1** - **Tracking of Spermatozoa and motility testing** - **initial optimisation**

*Introduction*

The analysis of spermatozoa motility in human semen samples is an important task in male fertility treatment. Few attempts were made to solve this task automatically using computer vision algorithms, however, none of them showed high enough accuracy and reliability. Hence, this analysis is still being performed manually by qualified technicians which implies high test costs and inaccuracy due to interpersonal variety in technicians performance.

In this work, we solve the problem of sperm motility measurement in 3 stages:

- Stage 1: Detecting the sperm cells on each frame separately.
- Stage 2: Tracking the sperm cells by assigning them a unique id that does not change throughout the video.
- Stage 3: Computing and analysing the sperm cells velocity.

YOLO approach was used for object detection (Redmon et al., 2015, "You only look once: Unifid, realtime object detection") and StrongSORT approach was used for object tracking (Du et al., 2022, "Strongsort: Make deepsort great again") together with OSNet (Zhou et al., 2019, "Omni-scale feature learning for person re-identification").

Previous studies of sperm analysis (e.g. Hicks et al., 2019, "Machine learning-based analysis of sperm videos and participant data for male fertility prediction") show great potential of machine learning approach to the problem, however they are all based only on raw, unlabelled video frames and do not leverage the information of bounding boxes manually annotated by human raters.

*Approach*

For object detection and object tracking, YOLOv5, StrongSORT and OSNet were used. This study found that adjusting the training parameters and the train-validation split of the dataset are critical for the final results of the model.

*Results - Train-validation split of the dataset*

The task organizers provided a sample code for object detection using YOLOv5 on the task dataset. In the solution, the full dataset was split by taking 16 full videos as a training set and the reminding 4 videos as a validation set. We found that in this setup the network learns well the training set, while for the validation the precision stays at the level of $mAP_{0.5} \sim 14\%$, $mAP_{0.5:0.95} \sim 4\%$. This means that the differences between the videos from the training set and the validation set are large and the network does not generalize well beyond the training set.

The other split we tried was to take every 5th frame (frame 0, 5, 10, 15, ...) as a validation set while keeping the rest as a training set. In this setup the network quickly overfit and reaches the precision of $mAP_{0.5} \sim 99.5\%$, $mAP_{0.5:0.95} \sim 90\%$ on the validation set. This means that the validation set is too similar to the training set and we are not able to verify how much the network generalizes beyond the training set and hence we cannot optimize the training parameters.

Finally, a training set was constructed from the first 24 s of each video and the validation set from the last 6 s of each video. In this setup the network achieved the accuracy of $mAP_{0.5} \sim 91\%$, $mAP_{0.5:0.95} \sim 66\%$ on the validation set. In this approach the network has a chance to learn on each of the different videos and at the same time the validation set is independent enough that the training parameters can be adjusted and their impact on the precision on the validation set can be analysed.

*Results - Confidence threshold for object detection*

The default confidence threshold (conf_thres) is set to 0.25 for object detection with YOLOv5. In this set up, the trained model detects more sperm per frame that it should, according to a manual assessment - it returns on average 78 sperm per frame in the test set, while there is only 22 sperm per frame in the labelled training set. The inventors have manually estimated the actual number of sperm in the test videos and compered it to the average number of sperm returned by the model for a few different thresholds and found out the most accurate number of sperm per frame are returned for threshold of 0.75. The average number of sperm detected for different thresholds are presented in Table 1.

Table 1. Average number of sperm detected per frame in the test videos for different confidence thresholds.

| conf_thres | avg. number of cells in the test videos |
|---|---|
| 0.25 | 78 |
| 0.50 | 67 |
| 0.70 | 44 |
| 0.75 | 31 |
| 0.80 | 17 |

*Results - Computing the motility of sperm*

To compute the motility of sperm, we use a novel approach of comparing the average (vector) velocities of cells for two different sampling intervals: $T_{long}$ and $T_{short}$. The approach is based on the observation, that progressive sperm have high velocity, independent of sampling interval while non progressive sperm have high velocity when sampling on short interval but low velocity when sampling on longer intervals. We determine the motility of a sperm by comparing its two average velocities $v_{long}$, $v_{short}$, and comparing them with two threshold velocities $v_{imm}$ and $v_{prog}$. The exact formula is as follows:

$$motility = \begin{cases} immotile & if \quad v_{short} \leq v_{imm}, \\ nonprogressive & if \quad v_{short} > v_{imm} \wedge v_{long} \leq v_{progr}, \\ progressive & if \quad v_{short} > v_{imm} \wedge v_{long} > v_{progr}. \end{cases}$$

We found that the most accurate results are obtained for $T_{short}$ = 5/50 s, $T_{long}$ = 20/50 s, $v_{imm}$ = 0.003$v_{max}$ and $v_{prog}$ = 0.01 $v_{max}$, where $v_{max}$ is the maximum sperm velocity measured at the 1/50 s sampling intervals.

*Results- Finding the fastest sperm*

The best accuracy in determining the fastest sperm was achieved by comparing the average velocity computed at 4 s sampling intervals. Moreover, to filter out the outliers coming from wrongly tracked sperms (the same sperm id jumping between two different sperm cells), the cells with standard deviation of the average velocity higher than 0.05 were removed from analysis.

*Results & analysis*

For computing and analysing the sperm cell velocity, the performance of the model was evaluated using the HOTA metric (Luiten et al., 2020, "A higher order metric for evaluating multi-object tracking"). This metric combines two components: Localization Accuracy and Association Accuracy and therefore can serve a single number to quantify the performance of both, detection and tracking parts of the workflow. The detailed HOTA numbers for detecting normal sperm are presented in Table 2.

Table 2. $HOTA_{AUC}$ metrics for detecting normal sperm in the test set of 5 videos.

| seq | $HOTA_{AUC}$ |
|---|---|
| 66 | 23.8% |
| 68 | 24.6% |
| 73 | 29.0% |
| 76 | 37.6% |
| 80 | 28.6% |
| combined | 28.3% |

Detection of sperm velocity was further evaluated by measuring different statistical errors between the predicted and ground-truth distribution of progressive/non-progressive/immotile sperm. The results are presented in Table 3

Table 3. Errors in predicting the progressive/non-progressive/immotile distribution.

| variable | mean absolute error | mean squared error | root mean squared error | root squared log error | median absolute error |
|---|---|---|---|---|---|
| progressive % | 15.6 | 295 | 17.2 | 1.36 | 17 |
| non progressive % | 8.6 | 84 | 9.2 | 0.26 | 10 |
| immotile % | 22.2 | 551 | 23.5 | 0.24 | 23 |
| average % | 155 | 310 | 16.6 | 0.62 | 17 |

**Example 2** - **Motility determination comparison with known point of care device**

A semen sample was analysed for motility parameters using the system and method of the present invention and using the device of as described in WO2019048018 A1 (available for purchase under the name ExSeed Home Sperm Test Kit).

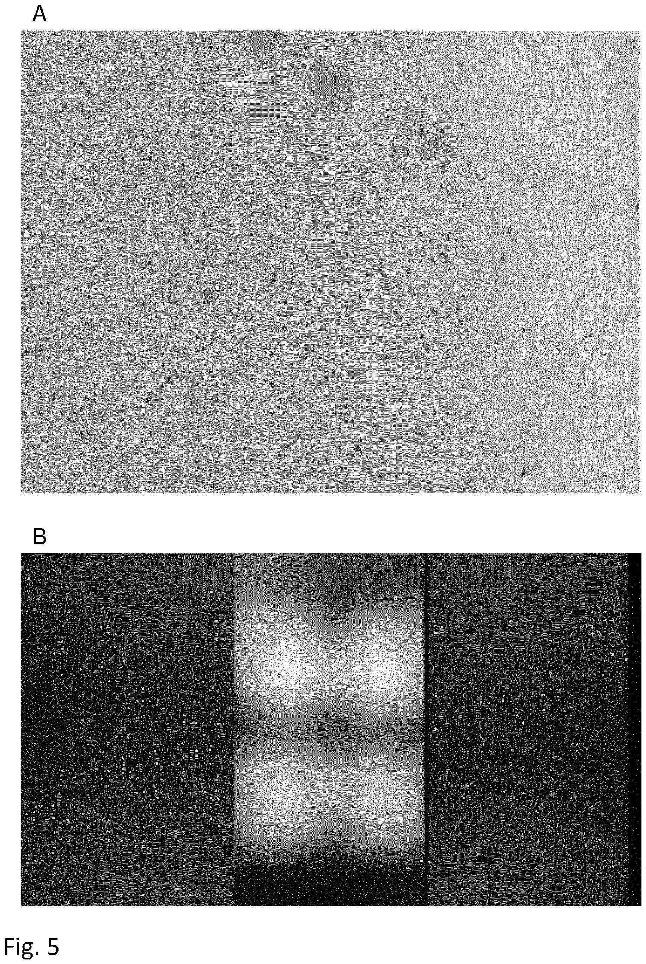

Fig. 5. Shows an exemplary image (or frame) taken using the ExSeed test kit (B) and the device of the present invention (A).

The ExSeed test kit determines the sperm motility as a single number M between 0% and 100% indicating the percentage of sperm cells that have moved. Based on this number, the sample is determined as having either low motility, moderate motility or high motility sperm.

In contrast, the present invention measures precisely velocity of each sperm cell (which is possible due to superior quality of the sample image obtained) and categorizes the velocity into one of 4 categories of sperm movement as recommended by the WHO manual (section 2.4.6.1). The motility determination method is as described in Example 3. This categorization is shown in Table 4 below.

Table 4. Categories of cell motility.

| velocity [$\mu$m/s] | | | |
|---|---|---|---|
| no active tail movements | < 5 $\mu$m/s | between 5 and 25 $\mu$m/s | $\geq$ 25 $\mu$m/s |
| immotile | non-progressive | slowly progressive | rapidly progressive |

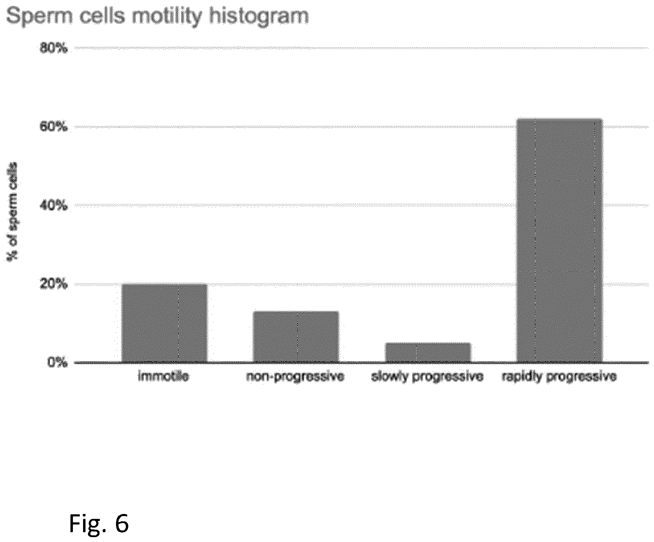

Additionally, the present invention performs velocity measurements for each sperm cell separately, and the aggregated result is presented as a histogram of sperm cell velocities. An exemplary histogram is shown in Fig. 6.

**Example 3** - **Methods for determination of motility, morphology, vitality, pH, total cell count and concentration**

*Motility*

The workflow for object detection and tracking is visualised in Fig. 3.

Step 1: Each frame is analyzed by the object detection algorithm. Output of stage 1 are "bounding boxes": coordinates of rectangles surrounding the heads of detected sperm cells. Typically, object detection is performed using Deep Learning models such as DeepTrack, YOLOor Mask-RCNN. Step 2: The coordinates of the sperm cells detected in step 1 are analyzed frame by frame to "track" each cell - assign each detection a "sperm id" - a single number that identifies the same cell on each frame where the cell occurs. This allows us to determine the "trajectory" of each sperm cell - a list of positions of the cell on every frame. The object tracking task is typically solved using Deep Learning models such as DeepSORT or StrongSORT.

Step 3: For each sperm cell $i$ and each time $t$, the displacement $\Delta x_i(t)$ is computed as the Euclidean distance between its positions in moments $t$ and $(t+ 1s)$. Then the average velocity of sperm $i$ is computed as:

$$v_i = \frac{1}{T-1s} \sum_{t=0}^{T-1s} \frac{\Delta x_i(t)}{1s}$$

where T is the length of the recording.

Each sperm is classified into one of 4 motility categories as shown in Table 4.

*Morphology and vitality*

Step 1: Similarly to Step 1 in motility assessment, each frame is analyzed by the object detection algorithm.

Step 2: For each detected sperm, the image classification algorithm is run to classify each detected sperm into one of the few possible classes. Image classification is typically done using Deep Learning models such as ResNet, EfficientNet, Inception.

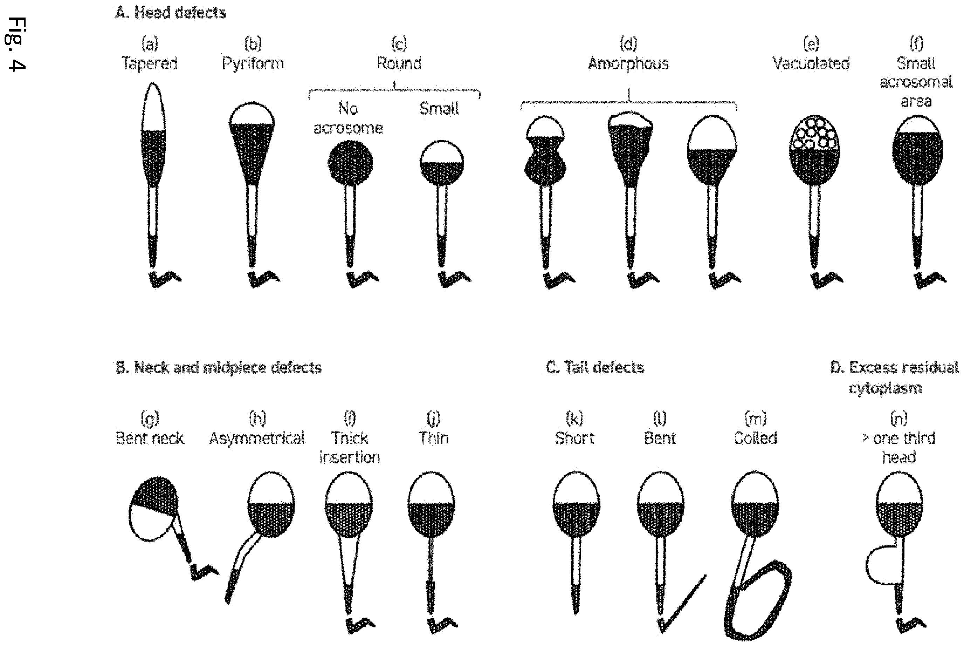

Morphology: For morphology, either 2 (normal/abnormal) or more classes are defined. The classes can span different defect groups (head defects, neck and midpiece defects, tail defects,excess residual cytoplasm) or be the 17 abnormal forms as shown in Fig. 4, or consist of some subgroups of it. The result of the assessment is the distribution of normal and abnormal sperm (for example: 24% normal, 76% abnormal) or more detailed distribution on all classes used in image classification.

Vitality: For vitality, the semen sample is mixed with a reagent that stains the sperm heads according to their vitality. Typically, it is Eosin Y which stains dead sperm heads red or pink and leaves live sperm heads white. The image classification model classifies each sperm cell from the stained sample into one of the two classes: dead or alive. The result of the assessment is the percentage of live sperm.

*pH*

In this assessment the semen sample is mixed with a pH indicator (for example Bogen Universal Indicator) which stains the semen into different colors depending on pH. Then, the image classification algorithm is run to classify the image into one of the classes corresponding to different colors and hence corresponding to different pH. The image classification task is typically solved using Deep Learning models such as ResNet, EfficientNet or Inception.

*Sperm count/concentration*

Step 1: Object detection is performed on each frame, similarly to motility and morphology/vitality. Step 2: The number of detections on a single frame is divided by the volume of the observed part of the sample to compute the sperm concentration. The volume of the observed part of the sample is computed as the thickness of the sample layer (fixed by the known height of the cell chamber) multiplied by the area visible by the device camera.

The concentration is averaged over all recording frames.

The total sperm count is computed as the sperm concentration multiplied by the volume of the ejaculate (retrieved from the scale on the collection cup).

## Claims

1. A method for point-of-care determination of at least three parameters of cells contained in a biological sample, preferably semen, wherein the method comprises the steps of:

   (a) combining (i) a first portion of the biological sample with a vitality reagent; and (ii) a second portion of the biological sample with a pH reagent;
   (b) recording with a camera (i) a first video of the first portion of the biological sample; (ii) a second video of the second portion of the biological sample; and (iii) a third video of a third portion of the biological sample; and
   (c) analysing the first, second, and third recorded videos of the biological sample to determine at least three parameters of the cells in the biological sample, wherein the at least three parameters are selected from morphology, vitality, pH, total count, concentration and motility.

2. The method of claim 1, wherein the method determines at least four, at least five, or at least six parameters of cells, preferably wherein the method determines at least six parameters of cells, wherein the parameters are morphology, vitality, pH, total count, concentration and motility.

3. The method of claim 1, wherein the at least three parameters are pH, vitality and motility.

4. The method of claim 2, wherein the vitality is determined from the first video, the pH is determined from the second video, and morphology, motility, total count and concentration are determined from the third video, optionally wherein morphology, motility, total count and concentration are further determined from the first video and the second video.

5. The method of any one of claims 1-4, wherein the step of analysing the first, second and third recorded videos is performed on an electronic apparatus.

6. The method of any one of claims 1-5, wherein the method further comprises applying (i) the first portion of the biological sample to a first location on a solid support, (ii) the second portion of the biological sample to a second location on a solid support, and (iii) the third portion of the biological sample to a third location on a solid support.

7. A system for point-of-care analysis of at least three parameters of a cell contained in a biological sample, wherein the biological sample is preferably semen, the system comprising:

   a. a device comprising:

   (i) a camera configured to record at least three videos of the biological sample;
   (ii) an optical system comprising at least one lens, wherein the optical system is configured to magnify an image of the biological sample; and
   (iii) at least one processor communicatively connected to the camera, the at least one processor being configured to:

a) receive the at least three recorded videos of the biological sample captured by the camera;

b) transmit, preferably via a Wi-Fi module, the at least three recorded videos of the biological sample to an electronic apparatus;

b. the electronic apparatus configured to receive the at least three recorded videos of the biological sample, and to:

A.

    i. identify a cell in a first frame of the first of the at least three recorded videos;

    ii. identify the cell in a second frame of the first of the at least three recorded videos;

    iii. identify the cell in a third frame of the first of the at least three recorded videos;

    iv. obtain (i) an average velocity of the cell based on the first, second and third frames of the first of the at least three recorded videos; and

    v. return distribution of motility of the cell in the biological sample, wherein the distribution of motility is obtained from the average velocity of the cell; and

B.

    i. identify a cell in the first frame of the first of the at least three recorded videos to obtain an image of the cell;

    ii. apply a machine-learning classification model to the image of the cell;

    iii. return the morphology of a cell, wherein morphology of the cell categorizes the cell as normal or abnormal, optionally wherein the system returns a category of morphology defects;

wherein the at least two of the at least three parameters are morphology and motility, and at least one of the at least three parameters is selected from vitality, pH, total count, and concentration.

8. The system of claim 7, wherein the system determines at least six parameters, wherein the six parameters are motility, morphology, total count, concentration, vitality and pH.

9. The system of claim 8 or claim 9, wherein:

a. in A., a plurality of cells is identified in the first, second and third frame of the first of at least three recorded videos, the average velocity is obtained for each cell in the plurality of cells, and the distribution of motility is obtained from the average velocities of each cell; and/or

b. in B., a plurality of cells is identified in the first frame of the first of the at least three recorded videos, and the returned morphology of the cells determines the percentage of cells that are normal and abnormal; and/or

c. steps A and B are also performed in the second and third of the at least three recorded videos and the returned motility and morphology are determined based on the first, second, and third recorded videos.

10. A device for point-of-care analysis of a biological sample, preferably semen, in cooperation with an electronic device, wherein the device comprises:

(a) a port configured to receive a solid support containing a biological sample;

(b) a light source configured to direct a light beam onto the solid support received in the port;

(c) an optical system comprising at least one lens, wherein the optical system is configured to magnify an image of the biological sample contained in or on the solid support; and

(d) an image processing system comprising (i) a camera configured to record at least one, preferably at least three, video(s) of the magnified biological sample; and (ii) an image processor configured to transmit, preferably via a Wi-Fi module, the at least one, preferably at least three, video(s) of the magnified biological sample to the electronic device.

11. The system of any one of claims 7-9 or the device of claim 10, wherein the optical system:

a. comprises two lenses; and/or

b. is configured to magnify the image of the biological sample at least 100 times.

**12.** The device of claim 10 or claim 11, wherein the device is configured so that the solid support is movable or slidable inside the device.

**13.** The device of any one of claims 10-12, wherein the device further comprises means for adjusting focus of the image of the biological sample.

**14.** A kit for point-of-care analysis of the biological sample, the kit comprising:

(a) the system of any one of claims 7-9 or the device of any one of claims 10-13;
(b) a solid support; and, optionally,
(c) one or more reagents for combining with the biological sample.

**15.** Use of the system of any one of claims 7-9 or the device of any one of claims 10-13 for diagnosing a disease associated with or resulting from male infertility and/or in veterinary diagnostics.

Fig. 1

Fig. 2

Fig. 3

| sperm id | velocity [µm/s] | motility |
|----------|-----------------|----------|
| 1 | 3.8 | non-progressive |
| 2 | 27.4 | rapidly progressive |
| 3 | 0.0 | immotile |
| ... | ... | ... |

Fig. 4

A

B

Fig. 5

Fig. 6

Fig. 7

Europäisches
Patentamt

European
Patent Office

Office européen
des brevets

# EUROPEAN SEARCH REPORT

Application Number

EP 23 46 1675

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2018/181792 A1 (SHAFIEE HADI [US]) 28 June 2018 (2018-06-28) | 1,3,5,6, 10-15 | INV. G01N33/68 |
| Y | * paragraph [0064]; claims 18-23; figures 4,7,8 * | 2,4,7-9 | A61B5/00 A61B10/00 G01N15/1433 |
| X | US 2021/374952 A1 (SHAFIEE HADI [US] ET AL) 2 December 2021 (2021-12-02) | 1,2,5-7, 9-15 | |
| Y | * paragraphs [0006], [0031]; figure 2 * | 3,4,8 | |
| X | US 2017/329120 A1 (HSU CHENG-TENG [TW] ET AL) 16 November 2017 (2017-11-16) * paragraphs [0012], [0076], [0086]; figures 3,11,15-18 * | 1-15 | |
| Y | US 8 535 622 B2 (SHANY VERED [IL]; TAVORI ISAAC [IL]; LOTUS BIO NYMPHAEA LTD [IL]) 17 September 2013 (2013-09-17) * claims 1,6 * | 2-4,7-9 | |
| A | WO 2014/140070 A2 (VENTANA MED SYST INC [US]; HOFFMANN LA ROCHE [CH]) 18 September 2014 (2014-09-18) * the whole document * | 1-15 | |

| | |
|---|---|
| TECHNICAL FIELDS SEARCHED (IPC) | |
| G01N A61B | |

The present search report has been drawn up for all claims

| Place of search | Date of completion of the search | Examiner |
|---|---|---|
| Munich | 17 April 2024 | Pinheiro Vieira, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                             
& : member of the same patent family, corresponding document

EPO FORM 1503 03.82 (P04C01)

**EP 4 549 944 A1**

## ANNEX TO THE EUROPEAN SEARCH REPORT
## ON EUROPEAN PATENT APPLICATION NO.

EP 23 46 1675

This annex lists the patent family members relating to the patent documents cited in the above-mentioned European search report.
The members are as contained in the European Patent Office EDP file on
The European Patent Office is in no way liable for these particulars which are merely given for the purpose of information.

17-04-2024

| Patent document cited in search report | | | Publication date | Patent family member(s) | | | Publication date |
|---|---|---|---|---|---|---|---|
| US 2018181792 | A1 | | 28-06-2018 | AU | 2016284332 | A1 | 15-02-2018 |
| | | | | AU | 2019202840 | A1 | 16-05-2019 |
| | | | | CA | 2995815 | A1 | 29-12-2016 |
| | | | | EP | 3310898 | A1 | 25-04-2018 |
| | | | | ES | 2898508 | T3 | 07-03-2022 |
| | | | | US | 2018181792 | A1 | 28-06-2018 |
| | | | | US | 2019347466 | A1 | 14-11-2019 |
| | | | | WO | 2016209943 | A1 | 29-12-2016 |
| US 2021374952 | A1 | | 02-12-2021 | AU | 2019346843 | A1 | 27-05-2021 |
| | | | | CA | 3114584 | A1 | 02-04-2020 |
| | | | | CA | 3210746 | A1 | 02-04-2020 |
| | | | | EP | 3856889 | A1 | 04-08-2021 |
| | | | | US | 2021374952 | A1 | 02-12-2021 |
| | | | | WO | 2020068380 | A1 | 02-04-2020 |
| US 2017329120 | A1 | | 16-11-2017 | CN | 108474735 | A | 31-08-2018 |
| | | | | EP | 3244250 | A1 | 15-11-2017 |
| | | | | TW | 201741664 | A | 01-12-2017 |
| | | | | US | 2017329120 | A1 | 16-11-2017 |
| | | | | WO | 2017197072 | A1 | 16-11-2017 |
| US 8535622 | B2 | | 17-09-2013 | EP | 2421360 | A1 | 29-02-2012 |
| | | | | US | 2012052485 | A1 | 01-03-2012 |
| | | | | WO | 2010122562 | A1 | 28-10-2010 |
| WO 2014140070 | A2 | | 18-09-2014 | AU | 2014230809 | A1 | 23-07-2015 |
| | | | | CA | 2897614 | A1 | 18-09-2014 |
| | | | | CN | 105027164 | A | 04-11-2015 |
| | | | | EP | 2973396 | A2 | 20-01-2016 |
| | | | | JP | 6378701 | B2 | 22-08-2018 |
| | | | | JP | 2016517559 | A | 16-06-2016 |
| | | | | US | 2016019695 | A1 | 21-01-2016 |
| | | | | WO | 2014140070 | A2 | 18-09-2014 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5605803 A
- WO 2019048018 A1

**Non-patent literature cited in the description**

- WHO laboratory manual for the examination and processing of human semen. WHO, 2021
- **REDMON et al.** *You only look once: Unifid, realtime object detection*, 2015
- **DU et al.** *Strongsort: Make deepsort great again*, 2022
- **ZHOU et al.** *Omni-scale feature learning for person re-identification*, 2019
- **HICKS et al.** *Machine learning-based analysis of sperm videos and participant data for male fertility prediction*, 2019
- **LUITEN et al.** *A higher order metric for evaluating multi-object tracking*, 2020